(19) Europäisches Patentamt
European Patent Office
Office européen des brevets

(11) Publication number: **0 099 572**
**B1**

(12) # EUROPEAN PATENT SPECIFICATION

(45) Date of publication of patent specification: **06.09.89**

(21) Application number: **83107066.9**

(22) Date of filing: **19.07.83**

(51) Int. Cl.[4]: **C 07 C 69/145,**
C 07 C 67/343, C 07 C 69/24 //
C07C69/16, C07C67/293,
C07C21/14

(54) **Disproportionation of functional olefins.**

(30) Priority: **20.07.82 US 400188**
**10.05.83 US 490110**

(43) Date of publication of application:
**01.02.84 Bulletin 84/05**

(45) Publication of the grant of the patent:
**06.09.89 Bulletin 89/36**

(84) Designated Contracting States:
**AT BE CH DE FR GB IT LI LU NL SE**

(56) References cited:
**EP-A-0 057 736**
**FR-A-2 389 588**
**FR-A-2 499 558**
**US-A-4 083 995**

**CHEMICAL ABSTRACTS, vol. 89, no. 23, 4th December 1978, page 575, no. 196934n, Columbus, Ohio, USA: R. ROSSI: Stereoselective synthesis of (Z)-9-dodecen-1-ylacetate, the sex pheromone of some trotricoids moths" & CHIM. IND. (MILAN) 1978, 60(8), 652-3**

(73) Proprietor: **PHILLIPS PETROLEUM COMPANY**
**5th and Keeler**
**Bartlesville Oklahoma 74004 (US)**

(72) Inventor: **Banasiak, Dennis Stephen**
**4661 Clearview Drive,**
**Bartlesville Oklahoma 74003 (US)**

(74) Representative: **Dost, Wolfgang, Dr.rer.nat., Dipl.-Chem. et al**
**Patent- und Rechtsanwälte Bardehle-Pagenberg-Dost-Altenburg & Partner Postfach 86 06 20**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 099 572 B1

## Description

Background of the invention

This invention relates to the disproportionation of functional olefins and to the products resulting therefrom.

The disproportionation of olefins containing other functional groups is a very desirable technique for obtaining valuable chemicals that otherwise would require multistep preparations that are costly and often give products that are difficult to purify. The disproportionation of olefins containing ester, halide or ether groups is particularly interesting in that it provides a route to chemicals having potential application in insect control because of the ability of the compounds to disrupt the mating of specific insects.

It has been noted by those working in the area of insect pheromones that the microstructure ratio of functional olefins can often have a significant influence upon the effectiveness of a particular functional olefin as an insect control agent. Thus, while some insects are attracted to a trans isomer, others may be attracted to a cis isomer, and still others may be best attracted by a particular molar ratio of cis and trans isomers.

A particularly useful catalyst system for the production of functional olefins has been disclosed in U.S. 4,269,780, the disclosure of which is incorporated herein by reference. The present invention is based upon the discovery that by selecting the proper reactants, it is possible to vary the isomer ratio in the recoverable functional olefin disproportionation product.

Accordingly, the invention relates to a process of preparing an internally unsaturated olefin having an oxygen-containing functional group obtained by reacting

(1) a functional monoolefin having at least one terminal functional group selected from the formulas

$$-O-\overset{\overset{O}{\|}}{C}-R,\quad -\overset{\overset{O}{\|}}{C}-O-R,\ \text{and}\ -OR$$

wherein R is a hydrocarbyl group having 1 to 20 carbon atoms or in the case of the first formula hydrogen, or an omega-halo-or omega-ester- alpha-olefin, with

(2) a hydrocarbyl internal monoolefin having at least three carbon atoms in the presence of a catalyst composition comprising (a) at least one neutral carbene complex having the formula

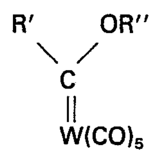

wherein R′ is selected from phenyl or an alkyl group having 1 to 10 carbon atoms and R″ is an alkyl radical having 1 to 10 carbon atoms; and

(b) a metal component comprising $WCl_6$ or a mixture of $SnCl_4$ and either $SiCl_4$ or $GeCl_4$, and then, in case of (1) being said omega -halo-alpha-olefin, converting the halide to an ester.

Whereas a similar process is known from EP—A 0 057 736, the process of the present invention distinguishes from the process of EP—A 0 057 736 i.a. that an internal hydrocarbyl monoolefin having at least three carbon atoms is used in the present process. The use of an internal olefin leads to a higher level of trans isomer in the reaction product compared to the use of an alpha-olefin.

Examples for functional olefins (1) include alkenyl acid esters, alkenyl esters, and alkenyl ethers. The preferred functional monolefins are those wherein the olefinic hydrocarbyl portion thereof has the formula

$$R'''-CH=CH(CH_2)_n-$$

wherein n is in the range of 2 to 20, preferably 2 to 10, R‴ is hydrogen or an alkyl radical having 1 to 20 carbon atoms. It is also within the scope of the present invention to use a functional monoolefin having two terminal functional groups of the types described above. Typically, the preferred functional monoolefins contain no more than about 30 carbon atoms per molecule.

The other olefin reactant in the inventive disproportionation is an internal hydrocarbyl monoolefin having at least three carbon atoms per molecule. It was found that when an internal monoolefin reactant is employed, as a general rule the level of trans isomer in the product is higher than that produced using an alpha-monoolefin capable of producing a compound having the same chemical formula. The currently preferred hydrocarbyl internal monoolefins are those having no more than about 20 carbon atoms per molecule.

If a symmetrical difunctional monoolefin reactant (1) is used that is substantially all in the trans form, the product obtained consists predominately of the trans isomer. By using such a technique, it has been found that it is possible to produce unsaturated functional olefins that are substantially all trans. This is

particularly surprising since disproportionation reactions are generally expected to provide a mixture of cis and trans isomers.

The catalyst system employed in this embodiment of the present invention comprises a neutral carbene complex and a metal component which acts as a promoter for the neutral carbene complex.

The molar ratio of the metal component (b) to the carbene complex (a) can vary over a wide range with varying effects upon yield and selectivity. Typically, the molar ratio of the metal component to the carbene complex is in the range of 1/1 to 500/1, preferably in the range of 2/1 to 25/1. The molar ratio of silica or germanium tetrachloride to tin tetrachloride is generally in the range of 0.5/1 to 20/1, most preferably about 2/1.

The amount of catalyst employed in the process of this invention can be expressed in terms of the molar ratio of olefin to carbene complex component. Generally, the molar ratio of the total stoichiometric amount of olefinic reactant to carbene complex component is in the range of 1/1 to 5000/1 and preferably from 50/1 to 2000/1. This term "total stoichiometric amount" is used herein to denote the moles of reactant that could theoretically react so as to distinguish from cases when two or more olefins are employed and one or more is used in a greater amount than will react. Thus, the amount of catalyst is generally based upon the amount of reactive olefins and not on excess olefin.

The disproportionation reaction of this invention can be carried out at temperatures between 35°C and 200°C. While lower temperatures can be used, the reaction rates are generally too low to be of interest. Temperatures above 200°C can be used, but excess decomposition of the reaction components can occur. The preferred reaction temperatures are from 50°C to 125°C.

The pressure during the disproportionation reaction can be from atmospheric to 34.5 MPa (5000 psig). Preferably, the pressure is from atmospheric to 6.9 MPa (1000 psig).

The disproportionation reaction can be carried out in the presence of diluents such as saturated hydrocarbons, e.g., hexane, octane, cyclohexane, aromatic hydrocarbons, e.g., benzene, toluene, or halogenated compounds, e.g., chlorobenzene, chloroform, methylene chloride or bromoform.

The amount of diluent can be expressed as a volume ratio of diluent to the olefin. Suitable volume ratios of diluent to olefin can be from 0/1 to 500/1 and preferably from 1/1 to 300/1.

The presence of oxygen and water has been found to be deleterious to the disproportionation reaction and should be substantially avoided during the reaction. Inert gases such as nitrogen, argon or helium can be used to maintain a dry, inert atmosphere during the reaction.

The metal compounds used as components of the catalyst system should be pure, free of oxygen or water, and free of any hydrolysis products. In general, the yield of disproportionation product decreases as the metal compound purity decreases.

The functional olefins used in the disproportionation reaction should be dry and free of polar materials such as carboxylic acids or alcohols. A purification step to remove impurities by such methods as filtering through silica gel or alumina and storing over molecular sieves or distilling from suitable drying agents is beneficial.

The reaction time period depends on the reaction temperature and pressure as well as on the nature of the particular catalyst system and olefinic reactant used. The reaction time is generally from 30 minutes to 120 hours.

In accordance with yet another embodiment of the present invention, there is provided a technique for producing internally unsaturated alpha esters from alpha-omega dienes. The technique involves the hydrohalogenation of the diene under conditions which favor maximum production of alpha-bromo-omega olefin.

Suitable dienes include those having 6 to 24 carbon atoms. Typical examples include 1,5-hexadiene; 1,6-heptadiene, 1,9-decadiene; 1,7-octadiene and 1,10-dodecadiene.

A preferred hydrohalogenation technique involves passing HBr through a solution of the diene. The solvent is preferably a nonpolar hydrocarbon or ether solvent having 4 to 12 carbon atoms per molecule such as diethyl ether, hexane or heptane. The optimum reaction temperature and times can vary depending upon the diene selected; however, generally the bromination is conducted at a temperature in the range of −10°C to 30°C and the reaction is terminated prior to the point where formation of dibromide or other undesired products such as oligomers begins to accelerate.

For best results, the hydrohalogenation is conducted in the presence of a small but effective amount of a free radical initiator such as metachloroperbenzoic acid or other peroxide catalysts such as aroyl peroxides, aryl peroxides, alkyl peroxides, hydroperoxides, and ketone peroxides. Other examples of typical free radical initiators include the azo type free radical initiators described in Encyclopedia of Polymer Science and Technology, Vol. 2, page 278 et seq., 1965 or any of the peroxide type free radical initiators described in the same publication at Vol. 9, page 814 et seq., 1968. Illustrative of azo type free radical initiators are 2,2′ - azobis(aliphatic nitriles) such as 2,2′ - azobisisopropionitrile or 2,2′ - azobisisobutyronitrile, 2,2′ - azobishexanonitrile, and azobisalkanes such as 1,1′ - azobisbutane, 1,1′ - azobishexane, and 1,1′ - azobisoctane. Illustrative of peroxide type free radical initiators are alkyl-aromatic peroxides such as dicumyl peroxide; dialkyl peroxides such as di - t - butyl peroxide, diisobutyl peroxide, diisopropyl peroxide, and diisohexyl peroxide; diperoxy ketals such as 2,2′ - bis(t - butyl - peroxy) butane and n-butyl 4,4′ - bis(t - butylperoxy) valerate; diacyl peroxides such as dibenzoyl peroxide, diacetyl peroxide, dilauroyl peroxide, and dipropionyl peroxide; peroxy esters such as t-butyl peroxypivalate,

t-butyl peroxyacetate, and t-butyl peroxybenzoate; dialkyl peroxydicarbonates such as diisobutyl peroxydicarbonate, and dihexyl peroxydicarbonate; ketone peroxides such as methyl ethyl ketone peroxide, and cyclohexanone peroxide; and hydroperoxides such as cumene hydroperoxide and t-butyl hydroperoxide.

The resulting alpha-olefin-omega-halide can then be converted into an internally unsaturated olefin by cross-disproportionation with an alpha or internal hydrocarbyl olefin of the type described previously in this disclosure. Any suitable catalyst system could be employed. Examples of suitable disproportionation catalyst system include the neutral carbene catalyst systems described previously in this disclosure. Other examples of suitable catalysts are the $WCl_6$— tetraalkyl tin catalyst systems, such as the tungsten hexachloride—tetrabutyl tin catalyst system.

The resulting internally unsaturated halide compound can then be readily converted to the ester by a nucleophilic displacement reaction involving the halide and an alkali metal ester, such as sodium acetate or sodium formate. Preferably, such reactions are carried out in the presence of dimethylformamide or a phase transfer agent.

In an alternative embodiment, one can first convert the alpha-olefin-omega-halide to an alpha-olefin-omega-ester by such a nucleophilic displacement reaction and then disproportionate the alpha-olefin-omega ester with a hydrocarbyl olefin. Again any suitable catalyst can be employed. The currently preferred catalyst is, however, the carbene catalyst system described above. By using the carbene catalyst system one can take advantage of the fact that the cis to trans ratio in the product can be controlled to some extent depending upon whether the olefin used in the disproportionation is an alpha-olefin or an internal olefin. As indicated above, the alpha-olefin generally gives a higher level of cis isomer than the internal olefin.

The alternative techniques disclosed herein involving either the disproportionation of alpha-olefin-omega-esters or alpha-olefin-omega-halides, provides one with the ability to obtain a certain degree of control over the cis/trans content of the desired product. By using a disproportionation involving an internal olefin and the unsaturated ester or olefins in general and the unsaturated halide, one can obtain a product having a typically thermodynamic sterochemical isomer ratio. The disproportionation of an alpha olefin and the unsaturated ester, on the other hand, allows one to obtain a product in which the ratio of trans to cis isomer is much lower than that of the typical thermodynamic isomer mixture.

The above-described processes followed by traditional chemical transformation steps are particularly useful for forming compounds that either correspond to or are mimics or analogs for the active compounds occurring in the natural pheromone composition of many insects. Examples of the compounds that can be produced according to the present invention which are known to be useful in insect control are Z - 13 - octadecen - 1 - ol, Z - 5 - dodecen - 1 - ol formate, Z and E - 7 - dodecen - 1 - ol formates, Z - 9 - tetradecene - 1 - ol formate, Z - 11 - hexadecen - 1 - ol formate, Z - 4 - decen - 1 - ol acetate, Z - 5 - decen - 1 - ol acetate, Z and E - 5 - decen - 1 - ol acetate, Z - 5 - undecen - 1 - ol acetate, Z-7 and Z-8 undecen-1-ol acetate, E and Z - 5 - dodecen - 1 - ol acetate, E and Z - 7 - dodecen - 1 - ol acetate, E and Z isomers of 7, 8, and 9 dodecen - 1 - ols, Z isomers of 7, 8, 9, 10 and 11 - tetradecen - 1 - ols, E isomers of 9 and 11 - tetradecen - 1 - ol, Z and E 11 - hexadecen - 1 - ol, E - 6 - tetradecen - 1 - ol acetate, E - 7 - tetradecen - 1 - ol acetate, E - 9 - tetradecen - 1 - ol acetate, Z - 9 - hexadecen - 1 - ol propionate, Z - 5 - tetradecenal, E - 4 - tridecen - 1 - ol acetate, E and Z isomers of 11 - hexadecenal, and Z - 9 - tetradecenal.

Some of the cis/trans isomer mixtures produced by the processes disclosed herein have been found to have utility in insect control which to the best of the inventor's knowledge would not have been predicted by those skilled in the art.

One particular example is 9-dodecenyl acetate obtained via a disproportionation of the types described herein. A prior art mixture of Z and E isomers having a ratio of 4/1 was known to be effective in disrupting the mating of the Western Pine Shoot Borer, *Eucosma sonomana*. In view of the fact that it is well recognized that different proportions of E and Z isomers can affect insects differently, it was surprising that the 9-dodecenyl acetate isomer mixture produced in accordance with this invention would also be effective since it contained a much larger proportion of the trans isomer than the prior art mixture. In view of the discovery that even a 2/1 trans to cis ratio mixture was effective, it is considered reasonable to assume that a disruptive effect of varying degree will also be observed for 9-dodecenyl acetate containing isomer ratios greater than 0.25/1 up to about 3/1.

Another isomer mixture found to have disruptive activity was the 9-tetradecenyl formate produced by the present invention. While the cis isomer has been recognized as having a disruptive effect upon certain insects, to the best of the inventor's knowledge there has been no indication that a cis/trans isomer mixture of that compound would be effective. In view of the results disclosed below, it is now however considered that a disruptive effect will be obtained on *Heliothis spp.* with trans to cis mixtures of 9 - tetradecenyl formate, particularly those mixtures having trans to cis ratios in the range of 1/1 to 6/1, and more particularly 2/1 to 5/1. (It is noted that another nomenclature for the 9 - tetradecenyl format is 9 - tetradecen - 1 - ol format. Similar nomenclature exists for the other products obtained in accordance with this invention. As a matter of convenience, the shortened nomenclature has been used throughout to refer to the internally unsaturated terminally functional products of this invention.)

Still another isomer mixture found to have disruptive effect upon the *Heliothis zea* is that of 9 - hexadecenyl alcohol.

Still yet another isomer mixture found to have disruptive effect upon the *Heliothis* species is that of 9 - tetradecenal. While the cis form of this compound has been recognized as useful in dealing with *Heliothis spp.*, to the best of the inventor's knowledge there has not heretofore been any recognition of the fact that such a cis/trans isomer mixture could be effective as a disruptant.

A still better understanding of the present invention and its advantages will be provided by the following examples.

The non-functional and functional olefins used in the following examples were commercial materials which were usually distilled, contacted with silica gel or alumina and stored over 4 A molecular sieves. Diluents were usually distilled from the appropriate drying agent and stored over 4 A molecular sieves. The various metal halides and organometallics were commercial materials which were normally used without further purification, but with careful exclusion of moisture and oxygen. In several cases, fresh reagents were found to give better disproportionation results than older reagents which are believed to have changed in chemical composition, for example by hydrolysis during aging. During the reaction evaluations, it was found that the careful exclusion of oxygen and moisture from the reaction system was extremely important for successful disproportionation results.

The carbene complexes were prepared by published procedures. Typically, they were prepared by reacting the appropriate metal carbonyl with an organolithium compound and then adding a trialkyloxonium tetrafluoroborate. For example, tungsten hexacarbonyl was reacted with phenyllithium and was then reacted with trimethyloxonium tetrafluoroborate to form (methoxyphenylcarbene)penta-carbonyltungsten(O)

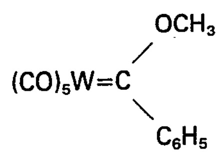

The carbene complexes were stored in a desiccator in a freezer before use in a reaction.

Some of the runs in the following examples were carried out in 280 ml (10 oz.) or 900 ml (32 oz.) beverage bottles equipped with a magnetic stirrer, a self-sealing elastomeric cap liner, and a three-hole crown cap. The liquid reaction components were charged to the dried, nitrogen flushed bottle by syringe through the cap. Solids were added to the bottle before attaching the cap. The reaction mixture was heated to the desired temperature and stirred at the reaction temperature for the desired time period. Venting was accomplished by means of a syringe needle through the bottle septum. The needle is attached to an oil filled bubble-tube capped with a Drierite filled drying tube.

Some of the runs in the following examples were carried out in 300 mL or 1000 mL Hastelloy C magnedrive packless autoclave. A side port on each autoclave was equipped with a ball valve to allow passage of a syringe needle into the autoclave. In some runs the reactions were vented by manually opening a valve to allow the autoclave to vent to the desired lower pressure. In other runs, venting was accomplished by means of a variable pressure relief valve which was self-sealing if the pressure dropped below the set pressures.

The autoclave was sealed empty, purged with dry nitrogen and heated to 150°C or higher for at least one hour, then cooled under a nitrogen sweep to room temperature. The autoclave was left sealed under at least 207 kPa sample (30 psig) $N_2$ and left overnight (for convenience the reactor was dried the day before use). The next day a slight $N_2$ sweep was again started and the reagents added by syringe through a ball valve which was also the nitrogen vent. The order of reagent addition does not appear critical but the following order was utilized: 1) solvent; 2) olefins; 3) solution of carbene; and 4) solutions of cocatalysts. GC standards were added prior to the olefins or if the standard could not be tolerated by the catalyst, at the end of reaction. The ball valve was then closed and the reactor pressurized with nitrogen to the desired pressure. Heating by means of an external heater and stirring were then started. (Temperatures were controlled by means of either a Thermoelectric #400 TM or an Eurotherm™ Temperature Controller. Temperatures were determined with Type J thermocouples placed in the autoclave thermowell and were read from an Acromag™ Temperature Indicator. Pressure readings were made visually from gauges on the autoclave.) Heating and stirring were continued for 2 to 8 hours. Time of reaction was measured from the point reaction temperature was reached, thus, the overall time the reagents were heated in the reactor was somewhat longer than indicated. When the desired time of reaction was reached the heater was removed and compressed air was used to cool the exterior of the reactor. The reactor was then vented to atmosphere and opened. The reaction mixture was removed. Analysis and work up was the same as that of the beverage bottle reactions.

Reactions carried out at atmospheric pressure were run in an oven-dried 3-neck flask (1, 3, 5, and 12 liter flasks were employed). The flask was equipped with a magnetic or a mechanical stirrer, a dry ice or water condenser and a septum inlet. Once sealed, the reactor was flushed with a nitrogen flow and kept under a nitrogen atmosphere while the reagents (carbene plus metal halide catalyst components, solvent,

functional olefin reactant) were added. The flask was then heated to the desired reaction temperature, typically about 80°C, and introduction of the gaseous olefinic reactant was begun via the septum inlet.

Alternatively, the carbene plus metal halide catalyst components and solvent could be charged to the reactor first, then the olefinic reactant introduced and allowed to react for a period of time before the functional olefin reactant is introduced. In this manner, an alphaolefin reactant, such as 1-butene, can be converted, in situ, to an internal olefin (3-hexene) which typically gives a higher trans-content product when cross - disproportionation of a functional and non-functional olefin is carried out.

Yields were determined by gas chromatography (G.C.) employing internal standard. Yields are calculated based on the amount of functional olefin charged as that is generally the limiting reagent. A Perkin Elmer Sigma 3™ chromatograph with flame ionization detector was used. Either a 3 mm (1/8 in)×3 m (10 ft) 10% OV225™ on Chromosorb P™ or a 3 mm (1/8 in)×3m (10 ft) 10% Altech CS-8™ on Chromosorb W-AW™ column was employed. Cis-trans determinations were accomplished using a 6 mm ($\frac{1}{4}$ in)×4.9 m (16 ft) 15% Silar 10C™ glass column, and were confirmed by C-13 analysis of isolated product.

For product isolation, the reactor contents were diluted with solvent, then treated with enough 10% aqueous ammonium hydroxide to precipitate the transition metal complexes. The solution, normally yellow in color, was filtered. The organic layer was washed with water, and then with water saturated with sodium chloride, and dried over anhydrous magnesium sulfate. The dried organic layer was then filtered and solvent removed. The residue thus obtained was ready for distillation.

The Examples and Control Examples illustrate the invention.

Control Example 1

9-Tetradecenyl acetate from 1-hexene and 9-decenyl acetate

A series of runs were carried out for the preparation of 9-tetradecenyl acetate from 1-hexene and 9-decenyl acetate. Except as noted in Table I, the carbene employed was (methoxyphenylcarbene)pentacarbonyltungsten(O), and the metal halide catalyst component consisted of a mixture of $SnCl_4$ and $SiCl_4$. Reagent quantities employed, reaction conditions and reaction results are presented in Table I.

TABLE I

| Run # | 1-Hexene, mol | Acetate mol | Catalyst, mol | | Diluent, | mL | Time hrs. | Temp., °C | Press., kPa gauge (psig) | Yield | Trans/cis |
|---|---|---|---|---|---|---|---|---|---|---|---|
| | | | Carbene | $SnCl_4/SiCl_4$ | | | | | | | |
| 1 | 0.22 | 0.22 | 0.001 | 0.022/0.044 | $C_6H_5Cl$ | 70 | 4 | 80 | 552 (80) | 8.6 | 1.8 |
| 2 | 0.22 | 0.11 | 0.001 | 0.022/0.044 | $C_6H_5Cl$ | 90 | 4 | 82 | 552 (80) | 14.4 | 2.0 |
| 3 | 0.44 | 0.22 | 0.004 | 0.088/0.166 | $C_6H_5Cl$ | 180 | 4.5 | 82 | 35 (5) | 28.3 | 3.2 |
| 4 | 0.44 | 0.22 | 0.001 | 0.022/0.044 | $C_6H_5Cl$ | 90 | 4.5 | 84 | 69 (10) | 38.6 | 3.1 |
| 5 | 0.44 | 0.11 | 0.001 | 0.022/0.044 | $C_6H_5Cl$ | 90 | 4.5 | 85 | 21 (3) | 28.4 | 3.0 |
| 6 | 0.44 | 0.22 | 0.001 | 0.022/0.044 | $C_6H_5Cl$ | 90 | 4.5 | 82 | 69 (10) | 39.6 | 2.8 |
| 7 | 0.44 | 0.11 | 0.001 | $(WCl_6)$0.002 | Hexane | 115 | 4 | 104 | 414 (60) | 11.7 | 1.5 |
| 8 | 0.44 | 0.22 | 0.001 | 0.01/0.02 | $C_6H_5Cl$ | 100 | 4 | 80 | 138 (20) | 6.3 | 1.6 |
| 9* | 0.88 | 0.44 | 0.002* | 0.02/0.04 | $C_6H_5Cl$ | 200 | 5 | 79 | 35 (5) | 3.5 | 1.9 |
| 10** | 0.44 | 0.22 | 0.002** | 0.02/0.04 | $C_6H_5Cl$ | 100 | 5 | 78 | 35 (5) | 0.7 | n.d. |
| 11 | 1.78 | 0.89 | 0.0033 | 0.033/0.099 | Hexane | 200 | 5 | 79 | 69 (10) | 24.6 | 1.8 |
| 12 | 1.88 | 0.89 | 0.002 | 0.04/0.08 | $C_6H_5Cl$ | 160 | 4.5 | 77 | 35 (5) | 10.5 | 1.3 |
| 13 | 1.78 | 0.89 | 0.0022 | 0.022/0.044 | $C_6H_5Cl$ | 210 | 5 | 81 | 35 (5) | 18.8 | 1.5 |
| 14 | 0.44 | 0.22 | 0.001 | 0.01/0.02 | $C_6H_5Cl$ | 47 | 7 | 85 | 69 (10) | 29.5 | 1.7 |
| 15 | 0.44 | 0.22 | 0.001 | $(WCl_6)$0.002 | $C_6H_5Cl$ | 40 | 4 | 81 | 69 (10) | 36.5 | 2.3 |
| 16 | 0.89 | 0.44 | 0.002 | 0.02/0.04 | $C_6H_5Cl$ | 200 | 4.5 | 83 | 35 (5) | 30.7 | 1.8 |
| 17 | 0.11 | 0.056 | 0.00025 | 0.0025/0.005 | Hexane | 22 | 20 | 80 | — | 25.2 | 1.8 |
| 18 | 0.89 | 0.22 | 0.001 | 0.01/0.02 | Hexane | 50 | 5 | 78 | 207 (30) | 18.6 | 1.7 |
| 19 | 0.89 | 0.22 | 0.001 | 0.01/0.02 | $C_6H_5Cl$ | 50 | 5 | 76 | 414 (60) | 9.9 | 1.5 |

TABLE I (continued)

| Run # | 1-Hexene, mol | Acetate, mol | Catalyst, mol Carbene | Catalyst, mol $SnCl_4/SiCl_4$ | Diluent, | mL | Time hrs. | Temp., °C | Press., kPa gauge (psig) | Yield | Trans/cis |
|---|---|---|---|---|---|---|---|---|---|---|---|
| 20 | 0.89 | 0.44 | 0.002 | 0.02/0.04 | Hexane | 200 | 5 | 78 | 414 (60) | 14.4 | 1.7 |
| 21 | 1.78 | 1.78 | 0.004 | 0.04/0.08 | Hexane | 80 | 4 | 80 | 242 (35) | 15.1 | 1.7 |
| 22 | 0.88 | 0.44 | 0.001 | 0.01/0.02 | Hexane | 150 | 5 | 81 | 207 (30) | 15.6 | 1.7 |
| 23 | 1.78 | 0.89 | 0.004 | 0.04/0.08 | Hexane | 80 | 4 | 84 | 242 (35) | 16.1 | 1.8 |
| 24 | 0.22 | 0.11 | 0.001 | 0.01/0.02 | $C_6H_5Cl$ | 100 | 4 | 84 | 35 (5) | 39.1 | 2.8 |
| 25 | 0.22 | 0.11 | 0.001 | 0.01/0.02 | $C_6H_5Cl$ | 100 | 4 | 87 | 656 (95) | 24.0 | 2.5 |
| 26 | 0.22 | 0.22 | 0.001 | 0.01/0.02 | $C_6H_5Cl$ | 100 | 4.5 | 88 | 276 (40) | 12.7 | 1.7 |
| 27 | 0.44 | 0.11 | 0.001 | 0.01/0.02 | $C_6H_5Cl$ | 100 | 4 | 87 | 138 (20) | 36.0 | 2.8 |
| 28 | 0.22 | 0.11 | 0.0005 | 0.01/0.02 | $C_6H_5Cl$ | 90 | 4 | 81 | 69 (10) | 21.9 | 2.0 |
| 29 | 0.88 | 0.22 | 0.002 | 0.04/0.08 | $C_6H_5Cl$ | 140 | 4 | 79 | 414 (60) | 25.5 | 1.6 |
| 30 | 15.7 | 4.1 | 0.011 | 0.2/0.4 | $C_6H_5Cl$ | 500 | 42 | 70 | atm. | 22.8 | 1.3 |
| 31 | 0.5 | 0.05 | 0.0003 | 0.002/0.04 | $C_6H_5Cl$ | 50 | 4 | 101 | 483 (70) | 16.3 | 1.3 |
| 32 | 0.5 | 0.12 | 0.0005 | 0.011/0.022 | $C_6H_5Cl$ | 60 | 2 | 106 | 552 (80) | 11.0 | 1.6 |

*(ethoxyphenylcarbene)pentacarbonyltungsten(O).
**(methoxymethylcarbene)pentacarbonyltungsten(O).

The results in Table I demonstrate that the yield and trans/cis ratio of desired cross-disproportionation product is seen to vary as a function of 1) functional/non-functional olefin ratio (compare Runs 1, 2 and 4), 2) olefin/carbene ratio (compare Runs 12 and 14), 3) carbene/metal halide ratio (compare Runs 6 and 14), 4) the metal halide employed (compare Runs 7 and 20), 5) solvent employed (compare Runs 16 and 22, 20 and 21); 6) the amount of solvent employed (compare Runs 21 and 29); 7) reaction pressure (compare Runs 26 and 27).

Example 1

The 5-decene utilized in Examples 1 and 2 was prepared as follows:

An oven-dried 2-L, 3-neck round bottom flask was equipped with a magnetic stirrer and a reflux condenser. The condenser outlet was connected to a mercury filled bubble tube capped with a $CaSO_4$ drying tube. The open necks of the reaction flask were capped with septa. The reagents were charged via syringe through the septum, then the vessel was heated to 80°C for 24 hours.

In an exemplary reaction, 672 g (8 mol) of hexene were reacted in the presence of 0.0045 mol (methoxyphenyl)pentacarbonyltungsten(O), 0.10 mol $SnCl_4$ and 0.15 mol $SiCl_4$ with about 100 mL chlorobenzene as solvent. After 24 hours, reaction mixture was treated with aqueous ammonium hydroxide, extracted, and distilled. An isolated yield of 46% was obtained. The 5-decene had a trans to cis ratio of about 4:1.

9-Tetradecenyl acetate from 5-decene and 9-decenyl acetate

A series of runs were carried out for the preparation of 9 - tetradecenyl acetate from 5-decene having a trans/cis ratio of about 4:1 and 9-decenyl acetate. The carbene employed was (methoxyphenylcarbene)-pentacarbonyltungsten(O), and the metal halide catalyst component, unless otherwise noted, consisted of a mixture of $SnCl_4$ and $SiCl_4$. Reagent quantities employed, reaction conditions and reaction results are presented in Table II.

TABLE II

| Run # | 5-Decene, mol | Acetate, mol | Catalyst, mol | | Diluent, | mL | Time hrs. | Temp., °C | Press., kPa gauge (psig) | Yield | Trans/cis |
|---|---|---|---|---|---|---|---|---|---|---|---|
| | | | Carbene | $SnCl_4/SiCl_4$ | | | | | | | |
| 1 | 0.22 | 0.22 | 0.0005 | 0.01/0.02 | $C_6H_5Cl$ | 90 | 4.5 | 84 | 276 (40) | 20.5 | 5.0 |
| 2 | 0.22 | 0.22 | 0.001 | 0.01/0.02* | $C_6H_5Cl$ | 150 | 4 | 100 | 483 (70) | 23.7 | 6.0 |
| 3 | 0.55 | 0.55 | 0.0025 | 0.025/0.05 | $C_6H_5Cl$ | 325 | 4.5 | 101 | 345 (50) | 45.3 | 5.0 |
| 4 | 0.55 | 0.55 | 0.0025 | 0.025/0.05 | $C_6H_5Cl$ | 325 | 4 | 100 | 690 (100) | 59.2 | 5.1 |
| 5 | 1.11 | 1.11 | 0.0025 | 0.025/0.05 | $C_6H_5Cl$ | 125 | 5 | 95 | 897 (130) | 28.4 | 7.0 |
| 6 | 0.11 | 0.11 | 0.0005 | 0.01/0.02 | $C_6H_5Cl$ | 90 | 4.5 | 83 | 311 (45) | 30.0 | 4.7 |
| 7 | 0.22 | 0.22 | 0.001 | 0.01/0.02 | $C_6H_5Cl$ | 100 | 7 | 84 | 276 (40) | 12.9 | 5.3 |
| 8 | 0.22 | 0.22 | 0.005 | 0.01/0.01* | $C_6H_5Cl$ | 70 | 4 | 82 | 242 (35) | 9.3 | 11.1 |
| 9 | 0.56 | 0.56 | 0.0025 | 0.025/0.04 | $C_6H_5Cl$ | 315 | 4 | 80 | 414 (60) | 37.7 | 4.0 |
| 10 | 0.056 | 0.056 | 0.00025 | 0.0025/0.005 | Hexane | 22 | 20 | 80 | — | 45.7 | 5.0 |
| 11 | 0.42 | 0.42 | 0.002 | 0.02/0.04 | Hexane | 200 | 5 | 84 | 345 (50) | 38.0 | 5.0 |
| 12 | 0.11 | 0.11 | 0.0005 | 0.005/0.01* | $C_6H_5Cl$ | 100 | 4 | 102 | 345 (50) | 15.3 | 4.4 |
| 13 | 0.11 | 0.11 | 0.001 | 0.01/0.02 | Hexane | 125 | 4 | 84 | 242 (35) | 13.7 | 7.2 |
| 14 | 0.11 | 0.11 | 0.0005 | 0.005/0.01 | $C_6H_5Cl$ | 100 | 4 | 101 | 345 (50) | 43.6 | 4.9 |
| 15 | 0.22 | 0.22 | 0.0005 | 0.005/0.01 | Hexane | 75 | 5.5 | 98 | 557 (80) | 12.6 | 8.0 |

TABLE II (continued)

| Run # | 5-Decene, mol | Acetate, mol | Catalyst, mol | | Diluent, | mL | Time hrs. | Temp., °C | Press., kPa gauge (psig) | Yield | Trans/cis |
|---|---|---|---|---|---|---|---|---|---|---|---|
| | | | Carbene | $SnCl_4/SiCl_4$ | | | | | | | |
| 16 | 0.11 | 0.11 | 0.001 | 0.01/0.02 | $C_6H_5Cl$ | 125 | 5 | 95 | 518 (75) | 45.0 | 3.6 |
| 17 | 0.22 | 0.11 | 0.001 | 0.01/0.02 | Hexane | 125 | 4 | 81 | 276 (40) | 51.1 | 4.4 |
| 18 | 0.11 | 0.11 | 0.0005 | $(WCl_6)$0.01 | $C_6H_5Cl$ | 70 | 4 | 108 | 483 (70) | 33.6 | 5.8 |
| 19 | 0.42 | 0.42 | 0.002 | 0.02/0.04 | Hexane | 180 | 4.5 | 77 | 552 (80) | 40.5 | 4.4 |
| 20 | 1.0 | 1.0 | 0.0025 | 0.05/0.1 | None | | 20 | 80 | — | 23.0 | 5.0 |
| 21 | 0.4 | 0.1 | 0.0005 | $(WCl_6)$0.001 | $C_6H_5Cl$ | 50 | 4 | 106 | 138 (20) | 60.1 | >20.0 |
| 22 | 0.4 | 0.05 | 0.0003 | 0.003/0.005 | $C_6H_5Cl$ | 50 | 4 | 100 | 69 (10) | 82.6 | ~20.0 |

*$SnCl_4/GeCl_4$

The results in Table II demonstrate the higher trans/cis ratios obtained as a result of substituting the internal olefin, 5-decene, for the alpha-olefin 1-hexene of Example 1. A typical trans/cis ratio of 5:1 or greater is obtained upon reaction of 5-decene with 9-decenyl acetate.

Control Example 2

The 1,18-diacetoxy-9-octadecene utilized in Control Example 2 and Example 2 was prepared as follows:

The 300 mL autoclave was charged with 9-decenyl acetate (0.22 mol), (phenylmethoxycarbene)pentacarbonyltungsten(O) (0.001 mol), $SnCl_4$ (0.01 mol), $SiCl_4$ (0.02 mol) and 20 mL chlorobenzene. The autoclave was heated to 86° for 4 hours with continuous venting above 5 psig.

The reaction product, following removal of catalyst components, was vacuum distilled at 0.35 mm Hg. Material boiling between 205—230°C was collected and found to be pure 1,18 - diacetoxy - 9 - octadecene. The trans/cis ratio of distilled product was 1.7.

The crude reaction product was purified by standard crystallization techniques from ethanol solvent. The crystals so obtained were collected by filtration, analyzed, and found to be essentially pure trans - 1,18 - diacetoxy - 9 - octadecene.

9-Tetradecenyl acetate from 1-hexene and trans-1,18-diacetoxy-9-octadecene

A series of runs were carried out for the preparation of 9-tetradecenyl acetate from 1-hexene and the trans - 1,18 - diacetoxy - 9 - octadecene prepared as set forth in the preceding example. The carbene employed for the disproportionation of the acetate was (methoxyphenylcarbene)pentacarbonyltungsten(O), and the metal halide catalyst component consisted of a mixture of $SnCl_4$ and $SiCl_4$. The acetate product obtained was essentially all trans.

Example 2

9-Tetradecenyl acetate from 5-decene and trans-1,18-diacetoxy-9-octadecene

A series of runs were carried out for the preparation of 9-tetradecenyl acetate from 5-decene having a trans/cis ratio of about 4:1 and trans - 1,18 - diacetoxy - 9 - octadecene. Unless otherwise noted, the carbene employed was (methoxyphenylcarbene)pentacarbonyltungsten(O), and the metal halide catalyst component consisted of a mixture of $SnCl_4$ and $SiCl_4$. As in Control Example 2, essentially all trans product was obtained.

Example 3

9-Tetradecenyl acetate from mixed cis/trans-1,18-diacetoxy-9-octadecene

A series of runs were carried out for the preparation of 9-tetradecenyl acetate from 1-hexene or a trans/cis mixture of 5-decene and 1.7/1 trans/cis mixture of 1,18 - diacetoxy - 9 - octadecene. The carbene employed was (methoxyphenylcarbene)pentacarbonyltungsten(O), and the metal halide component consisted of a mixture of $SnCl_4$ and $SiCl_4$. Reagent quantities reacted, reaction conditions and reaction results are presented in Table III.

TABLE III

| Run # | Alkene, mol | Acetate, mol | Catalyst, mol | | Diluent, | mL | Time hrs. | Temp. °C | Press., kPa gauge (psig) | Yield | Trans/cis |
|---|---|---|---|---|---|---|---|---|---|---|---|
| | | | Carbene | $SnCl_4/SiCl_4$ | | | | | | | |
| 1[a] | 1-Hexane, 0.44 | 0.22 | 0.001 | 0.011/0.022 | $C_6H_5Cl$ | 120 | 4 | 92 | 207 (30) | 16.3 | 4.4 |
| 2[b] | 5-Decene, 0.44 | 0.22 | 0.001 | 0.011/0.022 | $C_6H_5Cl$ | 120 | 4 | 96 | 104 (15) | 3.1 | 5.5 |
| 3[b] | 5-Decene, 0.44 | 0.19 | 0.001 | 0.011/0.022 | $C_6H_5Cl$ | 120 | 4 | 91 | 104 (15) | 10.9 | 5.8 |

[a]Control
[b]Invention

The results in Table III demonstrate when trans/cis mixture 1,8 - diacetoxy - 9 - octadecene is employed, less trans isomer is obtained than when the octadecene reactant is substantially all trans. The trans level is slightly higher with the internal olefin reactant than the alpha-olefin reactant.

Control Example 4

9-Tetradecenyl acetate from 1-hexene and oleyl acetate

A series of runs were carried out for the preparation of 9-tetradecenyl acetate from 1-hexene and oleyl acetate. The carbene employed was (methoxyphenylcarbene)pentacarbonyltungsten(O), and the metal halide catalyst component consisted of a mixture of $SnCl_4$ and $SiCl_4$. Reagent quantities reacted, reaction conditions and reaction results are presented in Table IV.

TABLE IV

| Run # | 1-Hexene, mol | Acetate, mol | Catalyst, mol | | Diluent, | mL | Time hrs. | Temp., °C | Press., kPa gauge (psig) | Yield | Trans/cis |
|---|---|---|---|---|---|---|---|---|---|---|---|
| | | | Carbene | $SnCl_4/SiCl_4$ | | | | | | | |
| 1 | 0.44 | 0.11 | 0.0005 | 0.011/0.022 | $C_6H_5Cl$ | 60 | 4 | 109 | 276 (40) | 7.1 | 6.5 |
| 2 | 0.44 | 0.11 | 0.0005 | 0.011/0.022 | $C_6H_5Cl$ | 60 | 4 | 108 | 276 (40) | 8.1 | 2.1 |
| 3 | 0.44 | 0.11 | 0.0005 | 0.011/0.022 | $C_6H_5Cl$ | 60 | 2 | 107 | 1380 (200) | 7.8 | 1.6 |
| 4 | 0.44 | 0.22 | 0.0005 | 0.011/0.022 | $C_6H_5Cl$ | 30 | 2.25 | 105 | 1277 (185) | 3.8 | 1.5 |

On the average, a low trans/cis ratio product was obtained.

Example 4

9-Tetradecenyl acetate from 5-decene and oleyl acetate

A series of runs were carried out for the preparation of 9-tetradecenyl acetate from a trans/cis mixture of 5-decene and oleyl acetate. The carbene employed was (methoxyphenylcarbene)pentacarbonyl-tungsten(O), and the metal halide catalyst component consisted of a mixture of $SnCl_4$ and $SiCl_4$. Reagent quantities reacted, reaction conditions and reaction results are presented in Table V.

TABLE V

| Run # | 5-Decene, mol | Acetate, mol | Catalyst, mol | | Diluent, | mL | Time hrs. | Temp., °C | Press., kPa gauge (psig) | Yield | Trans/cis |
|---|---|---|---|---|---|---|---|---|---|---|---|
| | | | Carbene | $SnCl_4/SiCl_4$ | | | | | | | |
| 1 | 0.4 | 0.11 | 0.0005 | 0.011/0.022 | $C_6H_5Cl$ | 60 | 4.5 | 105 | 69 (10) | 6.3 | 2.1 |
| 2 | 0.4 | 0.11 | 0.0005 | 0.011/0.022 | $C_6H_5Cl$ | 60 | 4.5 | 92 | 690 (100) | 9.8 | 12.3 |

The results in Table V demonstrate if 5-decene is employed it is possible to obtain a higher trans/cis ratio than when oleyl acetate was reacted with 1-hexene.

Control Example 5

9-Dodecenyl acetate from 1-butene and 9-decenyl acetate

A series of runs were carried out for the preparation of 9-dodecenyl acetate from 1-butene and 9-decenyl acetate. The carbene employed was (methoxyphenylcarbene)pentacarbonyltungsten(O). Runs were made using each of the metal halide catalyst components, i.e., $WCl_6$, or a mixture of $SnCl_4$ and $SiCl_4$, or a mixture of $SnCl_4$ and $GeCl_4$. Trans/cis ratios of 1.2—5.0 were obtained with a 1.7 average.

Control Example 6

9-Dodecenyl acetate from 1-butene and oleyl acetate

A series of runs were carried out wherein 9-dodecenyl acetate was prepared from 1-butene and oleyl acetate. The carbene employed was (methoxyphenylcarbene)pentacarbonyltungsten(O), and the metal halide was $WCl_6$ or a mixture of $SnCl_4$ and $SiCl_4$. The trans/cis ratios were in the range of 1.1 to 1.2/1. For the best results in terms of yield excess 1-butene was needed.

Example 5

9-Dodecenyl acetate from 3-hexene and 9-decenyl acetate

Several runs were carried out for the preparation of 9-dodecenyl acetate from 3-hexene and 9-decenyl acetate. The carbene employed was (methoxyphenylcarbene)pentacarbonyltungsten(O), and the metal halide catalyst component consisted of a mixture of $SnCl_4$ and $SiCl_4$. Reagent quantities employed, reaction conditions and reaction results are presented in Table VI.

TABLE VI

| Run # | 3-Hexene, mol | Acetate, mol | Catalyst, mol | | Diluent, | mL | Time hrs. | Temp., °C | Press., kPa gauge (psig) | Yield | Trans/cis |
|---|---|---|---|---|---|---|---|---|---|---|---|
| | | | Carbene | $SnCl_4/SiCl_4$ | | | | | | | |
| 1 | 0.5 | 0.25 | 0.0005 | 0.005/0.01 | $C_6H_5Cl$ | 60 | 4 | 105 | 345 (50) | 7.8 | 5.2 |
| 2 | 1.61 | 0.25 | 0.002 | 0.01/0.02 | $C_6H_5Cl$ | 120 | 6 | 90 | 276 (40) | 69.1 | 5.4 |
| 3 | 1.61 | 0.25 | 0.002 | 0.01/0.02 | $C_6H_5Cl$ | 20 | 6 | 87 | 276 (40) | ~70.0 | 5.3 |
| 4 | 3.22 | 0.50 | 0.002 | 0.02/0.04 | $C_6H_5Cl$ | 220 | 5 | 92 | 276 (40) | — | 5.4 |
| 5 | 3.22 | 0.50 | 0.002 | 0.02/0.04 | $C_6H_5Cl$ | 220 | 6 | 84 | 242 (35) | 44.4 | 5.4 |

The results in Table VI demonstrate that the trans/cis ratio of 9-dodecenyl acetate typically obtained with the internal olefin, 3-hexene is significantly higher than that normally obtained with the terminal olefin, 1-butene.

Example 6

9-Tetradecenyl acetate and 9-undecenyl acetate from 9-decenyl acetate and 2-heptene

Several runs were carried out employing cis- or trans-2-heptene with 9-decenyl acetate or 1,18 - diacetoxy - 9 - octadecene having a trans to cis ratio of 1.7:1 in the presence of (methoxyphenylcarbene)-pentacarbonyl - tungsten(O) and $SnCl_4$ plus $SiCl_4$ as the metal halide catalyst component.

The trans/cis ratio of the 9-tetradecenyl acetate was in the range of 2.6—3.4, for the 9-undecenyl acetate it was in the range of 1.6 to 4.9. The 2-heptene produced slightly less trans isomer than an internal symmetrical olefin. The cis isomer of 2-heptene produced even less trans product than the trans isomer of 2-heptene.

Example 7

9-Tetradecenyl formate from 9-decenyl formate

Several runs were carried out for the preparation of 9-tetradecenyl formate from 9-decenyl formate and 1-hexene or 5-decene. The carbene employed was (methoxyphenylcarbene)pentacarbonyltungsten(O), and the metal halide catalyst component consisted of a mixture of $SnCl_4$ and $SiCl_4$. Reagent quantities employed, reaction conditions and reaction results are presented in Table VII.

TABLE VII

| Run # | Alkene, mol | Formate mol | Catalyst, mol | | Diluent, | mL | Time hrs. | Temp., °C | Press., kPa gauge (psig) | Yield | Trans/cis |
|---|---|---|---|---|---|---|---|---|---|---|---|
| | | | Carbene | $SnCl_4/SiCl_4$ | | | | | | | |
| 1[a)] | 1-Hexene, 0.22 | 0.11 | 0.002 | 0.02/0.04 | Hexane | 100 | 5 | 103 | 552 (80) | 7.4 | 1.8 |
| 2[a)] | 1-Hexene, 0.89 | 0.44 | 0.002 | 0.02/0.04 | $C_6H_5Cl$ | 200 | 4.5 | 78 | 34 (5) | trace | — |
| 3[b)] | 5-Decene, 0.26 | 0.11 | 0.001 | 0.01/0.02 | Hexane | 80 | 4 | 107 | 380 (55) | 3.0 | ~20.0 |
| 4[b)] | 5-Decene, 0.22 | 0.11 | 0.001 | 0.01/0.02 | $C_6H_5Cl$ | 125 | 5 | 102 | 345 (50) | 28.1 | — |
| 5[b)] | 5-Decene, 0.11 | 0.22 | 0.001 | 0.01/0.02 | Hexane | 150 | 4 | 101 | 311 (45) | trace | — |

[a)]Control
[b)]Invention

The results in Table VII demonstrate that the ratio of olefin reactants to the carbene catalyst can effect the yield. In the reactions in which the yield was significant, the alpha-olefin (1-hexene) gave 9-tetradecenyl formate with a trans/cis ratio of about 2/1 while the internal olefin (5-decene) gave 9-tetradecenyl formate with a trans/cis ratio of about 20/1.

Example 8

9-Tetradecenyl propionate from 9-decenyl propionate

Two cross-disproportionation reactions of 9-decenyl propionate were carried out employing 1-hexene and 5-decene. The carbene catalyst employed was (methoxyphenylcarbene)pentacarbonyltungsten(O), and the metal halide catalyst component consisted of a mixture of $SnCl_4$ and $SiCl_4$. The molar ratio of the 9-decenyl propionate to the carbene was 220/1. The molar ratio of carbene to $SnCl_4$ was 10/1 and the molar ratio of $SiCl_4$ to $SnCl_4$ was 2/1. The 5-decene was employed in a 1/1 molar ratio with the propionate. The 1-hexene was employed at a 2/1 ratio.

The trans/cis ratio obtained with the 1-hexene (control) was 1.8/1. The trans/cis ratio obtained with the 5-decene was 4.4/1.

Example 9

9-Tridecenyl acetate from 9-decenyl acetate

Two cross-disproportionation reactions of 9-decenyl acetate were carried out with 1-pentene or trans-4-octene. The carbene employed was (methoxyphenylcarbene)pentacarbonyltungsten(O), and the metal halide catalyst component consisted of a mixture of $SnCl_4$ and $SiCl_4$. The relative amounts of olefins, carbene, $SnCl_4$ and $SiCl_4$ were as in the preceding example.

Here again the alpha-olefin (control) produced a lower trans level than the internal olefin, namely 1.7/1 as compared to 12/1.

Example 10

9-Hexadecenyl acetate from 9-decenyl acetate

Several runs were carried out for the preparation of 9-hexadecenyl acetate from 9-tetradecenyl acetate and 1-octene or 7-tetradecene. The carbene employed was (methoxyphenylcarbene)pentacarbonyltungsten(O), and the metal halide component consisted of a mixture of $SnCl_4$ and $SiCl_4$.

The end product obtained with the terminal non-functional olefin (control) was relatively low in trans content about 1.8 trans to cis. The trans content of the product obtained with the internal olefin was not determined.

Example 11

4-Nonenyl acetate from 5-pentenyl acetate and 1-hexene or 5-decene

Two cross-disproportionation reactions of 5-pentenyl acetate were carried out with 1-hexene or 5-decene as the second reactant. The carbene employed was (methoxyphenylcarbene)pentacarbonyltungsten(O), and the metal halide catalyst component consisted of a mixture of $SnCl_4$ and $SiCl_4$.

Again, the internal olefin was found to give a much higher trans- content product than does the terminal olefin reactant (control) 2.1/1 vs. 6.5/1 for these two reactions.

Example 12

Methyl-10-pentadecenoate from methyl-10-undecenoate

Two cross-disproportionation reactions of methyl-10-undecenoate were carried out with 1-hexene or 5-decene as the second reactant. The carbene employed was (methoxyphenylcarbene)pentacarbonyltungsten(O), and the metal halide catalyst component consisted of a mixture of $SnCl_4$ and $SiCl_4$. Reagent quantities employed, reaction conditions and reaction results are presented in Table VIII.

TABLE VIII

| Run # | Alkene, mol | Undecenoate, mol | Catalyst, mol | | Diluent, | mL | Time hrs. | Temp., °C | Press., kPa gauge (psig) | Yield | Trans/cis |
|---|---|---|---|---|---|---|---|---|---|---|---|
| | | | Carbene | $SnCl_4/SiCl_4$ | | | | | | | |
| 1[a)] | 1-Hexene, 0.44 | 0.26 | 0.002 | 0.02/0.04 | $C_6H_5Cl$ | 100 | 4.5 | 83 | 35 (5) | 42.7 | 2.9 |
| 2[b)] | 5-Decene, 0.26 | 0.26 | 0.002 | 0.02/0.04 | $C_6H_5Cl$ | 100 | 5 | 82 | 414 (60) | 66.9 | 4.0 |

[a)]Control
[b)]Invention

The results again demonstrate that the internal olefin gives a product having a higher trans content than the alpha-olefin.

Example 13
Field tests with 9-dodecenyl acetate

Three adjacent test plots 610 m (2000 ft) wide and 1220 m (4000 ft) long in a ponderosa pine plantation in Oregon were used to evaluate a high trans-9-dodecenyl acetate product for its utility in controlling the Western Pine Shoot Borer, viz. *Encosoma sonomana*. The first plot was treated with commercially available pheromone for the Western Pine Shoot Borer, i.e., a 4/1 cis/trans 9-dodecenyl acetate at a rate of 9.9 g/ha (4 grams/acre) using commercially available dispensing means such as those disclosed in U.S. 4,160,335 and U.S. 4,198,782. The middle plot was treated in the same manner and at the same rate with 9-dodecenyl acetate prepared according to the present invention. The 9-dodecenyl acetate had a cis/trans ratio of 1/2. The third plot was not treated with any 9-dodecenyl acetate in order to serve as a control. The applications were carried out during the week of 3/30/81 and trap catches 4/27—4/30 and 4/30—6/2/81. Tree damage was observed at the end of the growing season in the fall of 1981. Trap catch data and tree damage results are presented in Table IX.

TABLE IX

| Total trap catches | Commercial (4/1) | Inventive (1/2) | Control |
|---|---|---|---|
| 4/3 —4/27 | 0 | 4 | 45 |
| 4/27—4/30 | 0 | 1 | 39 |
| 4/30—6/2 | 0 | 3 | 65 |
| % Tree damage | 21.5 | 24.0 | 39.8 |

The results presented in Table IX demonstrate the effectiveness of a 1/2 cis/trans ratio of 9-dodecenyl acetate for the disruption of mating of Western Pine Shoot Borer. In addition, the reduction of tree damage is comparable to the reduction obtained with the commercially available 4/1 cis/trans 9-dodecenyl.acetate. This inventive composition gave a 40% reduction in tree damage and the commercial composition a 46% reduction. It should be noted that % reduction in tree damage obtained in these tests is significant but even for the commercial product the results were not as good as results previously obtained with the product. The difference in the effectiveness of the commercial material is attributed to a problem observed in the application of the flake dispensing means. The same problem existed for the inventive composition. The problem involved the fact that a large portion of the flakes fell to the ground rather than becoming stuck in the trees.

Another evaluation was conducted of the 1/2 cis/trans product in 1982 employing improved application technology. In one plot 6,000 Hercon flake dispensing means were applied per acre to provide 9.9 g/ha (4 gm per acre) of the 1/2 isomer mixture. In another plot, 12,000 flake dispensing means were applied to provide 19.8 g/ha (8 gm per acre) of the 1/2 isomer mixture. The effects as compared to a control plot where no pheromone was applied are summarized in Table IXA.

TABLE IXA

| | 9.9 g/ha | 19.8 g/ha | Control |
|---|---|---|---|
| % Tree damage | 7.75 | 5.75 | 39.75 |
| Total trap catches | 0 | 0 | 239 |

The 4 gm/acre application gave an 81% reduction in tree damage and the 8 gm/acre application an 86% reduction. This is further evidence that the 1/2 isomer mixture is effective in disrupting the mating of *Eucosma sonomana*. In addition, these results are within the acceptable range for economical commercial application.

Example 14
Field tests—tetradecenyl formate

Fields of cotton in a late fruiting stage were used to evaluate the effectiveness of tetradecenyl formate in controlling Heliothis species. In this case, the 9-tetradecenyl formate was prepared from 9-tetradecenyl acetate (5/1 or 2/1 trans/cis content) rather than directly from 9-decenyl formate. In a typical process 21.1 g

(0.08 mol) of tetradecenyl acetate, 22.2 g (0.16 mol) $K_2CO_3$, 200 mL methanol, and 2 mL water were added to a 500 mL one neck round bottom flask. The mixture was stirred for two hours at room temperature. Then 200 mL of diethyl ether was added, and the mixture was filtered. The ether layer was washed with water, then saturated NaCl solution, then dried over $MgSO_4$. The dried solution was filtered and concentrated on a rotary evaporator. The product consisting of 9-tetradecenyl alcohol was then reacted with formic acid in benzene at gentle reflux (~80°C) for 24 hours. Then additional formic acid was added and the gentle reflux continued for another 24 hours. The 9-tetradecenyl formate was recovered by extraction.

Test plots measured 60 m (200 ft) long and 30 m (100 ft) wide and contained 60 rows. Plots were separated from each other by at least 60 m (200 ft) down row and by 30 m (100 ft) across rows. The test compound was laminated between 6×6 mm (¼"×¼") plastic flakes. Plots were treated with formate at the rate of 25 g/ha (10 grams/acre). In another plot flakes were used containing Z - 11 - hexadecenal, which has been reported as one of the components in the natural pheromone of *H. Zea* and *virescens*. The chemicals were applied on 8/12/81 and 9/1/81 and insects were monitored on the nights of 8/12—14 and 9/1—9, respectively. Separate traps were put out for *Heliothis zea* and *Heliothis virescens*. Trap catch data are presented in Table X.

TABLE X

| Total trap catches | 5/1 | 2/1 | Z-11-HDA | Control |
|---|---|---|---|---|
| 8/12—14 | | | | |
| *H. zea* | 5 | 2 | 0 | 87 |
| *H. virescens* | 30 | 34 | 45 | 213 |
| 9/1—9 | | | | |
| *H. zea* | 18 | 1 | 1 | 20 |
| *H. virescens* | 18 | 6 | 4 | 21 |

The results presented in Table X demonstrate the effectiveness, a 2/1 trans/cis ratio of 9-tetradecenyl formate for the disruption of mating of both *Heliothis* species. The 5/1 trans/cis ratio 9-tetradecenyl formate was also effective for disruption of mating *Heliothis* species.

Example 15
Field tests—9-tetradecenal

A 2/1 trans to cis ratio of 9-tetradecenal was evaluated in the same manner as was used in the preceding example. Z-9-tetradecenal which has been reported as a component in the natural pheromone of *H. Zea* was used for comparison. The 9-tetradecenal was prepared from 9-tetradecenyl alcohol prepared in the same manner as described in the preceding example. About 21 grams of the alcohol in a 50/50 weight ratio selection of dichloromethane was added dropwise to a flask charged with 43 g (0.2 mol) pyridinium chlorochromate, 20 g celite and 200 mL dichloromethane. The mixture was stirred at room temperature for about 2 hours after addition of the alcohol solution was complete. Then 200 mL of hexane was added and the mixture was filtered. The solvent was evaporated and the product vacuum distilled through a 30×2 cm (2 ft.×3/4 in.) packed column (Penn State stainless steel packing). A 36% yield of pure 9-tetradecenyl acetate was obtained.

The trap catch results are set forth in Table XI.

TABLE XI

| Total trap catches | Test plot | Z-9-TDA | Control plot |
|---|---|---|---|
| 8/12—14 | | | |
| *H. zea* | 1 | 9 | 87 |
| *H. virescens* | 44 | 24 | 213 |
| 9/1—9 | | | |
| *H. zea* | 13 | 1 | 20 |
| *H. virescens* | 2 | 3 | 21 |

The results presented in Table XI demonstrate the effectiveness of a 2/1 trans/cis ratio of 9-tetradecenal for the disruption of mating of the *Heliothis* species.

Example 16
Field test—9-hexadecenyl alcohol

The effect of a 2/1 trans/cis mixture of 9-hexadecenyl alcohol on *Heliothis* species was tested in the

same manner as described in Example XXI. Z-11-hexadecenyl alcohol was used in a comparison test. The alcohol was prepared by the hydrolysis of 9-hexadecenyl acetate. The results are shown in Table XII.

TABLE XII

| Total trap catches | Test plot | Z-11-HDOH | Control plot |
|---|---|---|---|
| 8/12—14 | | | |
| *H. zea* | 30 | 6 | 87 |
| *H. virescens* | 58 | 37 | 213 |
| 9/1—9 | | | |
| *H. zea* | 10 | 19 | 20 |
| *H. virescens* | 56 | 28 | 21 |

The results presented in Table XII demonstrate the effectiveness of a 2/1 trans/cis ratio of 9-hexadecenyl alcohol for the description of mating of *Heliothis zea*. Results for the description of *Heliothis virescens* are mixed, the 9-hexadecenyl alcohol showing disruptive activity in one test, but not in the other.

Examples 17a

Six hundred mL of a hexane solution of 1,7-octadiene (110 g, 1 mol) was added to a 2 L three-neck flask equipped with a bubble tube, a magnetic stir base, and a condenser and about 10 mg of metachloroperbenzoic acid was added. The flask and contents were cooled to about 0°C and HBr (80 g, 1 mol) was bubbled into the liquid in the flask over about 2 hours after being passed through a $CCl_4$ solution of phenol which removed any free bromine that might have been present in the HBr. By keeping the extent of diene conversion to less than about 50%, it was possible to obtain a 60% selectivity to 8-bromo-1-octene. The reaction product was washed with equal volume 10% aqueous $Na_2CO_3$ and then with an equal volume saturated aqueous NaCl solution. The recovered organic layer was dried over anhydrous $MgSO_4$ and then filtered. Distillation at reduced pressure was used to separate the 8-bromo-1-octene (bp 81 to 82°C at 11 mm Hg) from the dibromooctene.

Recovered 8-bromo-1-octene was then reacted with 1-hexene in an autoclave according to the general procedure set forth above in the presence of the homogeneous catalyst system of (methoxyphenylcarbene)pentacarbonyltungsten(O) (10 mL of *1M* catalyst in hexane) and a mixture of $SnCl_4$ (1 mL, 0.01 mol) and $SiCl_4$ (2.3 mL, 0.02 mol). The reaction was carried out for two hours at 80°C and about 40 psig. The reaction product included 7-dodecenyl bromide. From G. C. analysis it appeared that the trans/cis ratio of the bromide isomers was about 3/1.

In an oven dried one liter three-neck flask equipped with a magnetic stirrer and a reflux condenser, 46.5 g 7-dodecenyl bromide (0.19 mol) prepared as described above was combined with 20 g sodium formate (0.29 mol), and 300 mL dimethylformamide under a $N_2$ atmosphere. The mixture was heated to reflux and stirred for 4—5 hours. GC analysis showed that the reaction was complete. The reaction mixture was cooled and diluted with about 300 mL of ice water and extracted three times with equal volumes of ether. The ether layer was then washed with an equal volume $H_2O$, then two times with equal volumes of NaCl saturated $H_2O$, and finally the organic portion was dried over anhydrous $MgSO_4$. The resulting material was then distilled to obtain a product comprising 7-dodecenyl formate having a trans to cis isomer ratio of about 3/1 (bp 86 to 87°C at 0.5 mm Hg).

Example 17b

A hexane solution of 1,9-decadiene was reacted with HBr in the presence of metachloroperbenzoic acid in the same manner as the 1,7-octadiene was reacted in the preceding example 17a. The reaction product was also worked up as described above. The product boiling between 106 and 112°C at 7 to 8 mm Hg was essentially pure 9-decenyl bromide.

About 90 mL (0.5 mol) of 9-decenyl bromide, 100 mL (0.8 mol) of 1-hexene, 20 mL of an 0.1 *M* chlorobenzene solution of (methyloxyphenylcarbene)pentacarbonyltungsten(O) (0.002 mol) 2.8 mL (0.020 mol) $SnCl_4$, 5.2 mL (0.040 mol) $SiCl_4$ were combined and heated at about 80°C and about 120 psig for about 2 hours in 100 mL of chlorobenzene. The trans/cis isomer ratio in the 9-tetradecenyl bromide product was about 4/1 as shown by gas chromatography.

An analogous disproportionation reaction was carried out using 50 mL (0.23 mol) of 9-decenyl bromide, 87 mL (0.46 mol) of 5-decene, 10 mL of 0.1 M chlorobenzene solution of the carbene catalyst (0.001 mol), 1.4 mL (0.01 mol) of $SnCl_4$, and 2.6 mL (0.02 mol) of $SiCl_4$ in 100 mL of chlorobenzene. The trans/cis isomer ratio in the 9-tetradecenyl bromide was about 5/1 as shown by gas chromatography.

The 9-tetradecenyl bromide can be readily converted to 9-tetradecenyl acetate by reaction with sodium acetate in DMF as described in the preceding example.

The above results indicate that the selection of an internal or alpha-olefin reactant in the cross-disproportionation of the unsaturated bromide compounds does not have the same notable effect on

the trans/cis ratio as it has on those olefins containing ester groups. That is to say whereas internal and alpha-olefins produce quite different isomer ratios when reacted with unsaturated esters there is much less difference when an unsaturated bromide compound is substituted for the unsaturated ester.

## Claims

1. A process of preparing an internally unsaturated olefin having an oxygen-containing functional group obtained by reacting

(1) a functional monoolefin having at least one terminal functional group selected from the formulas

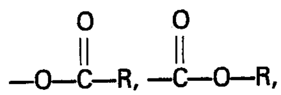, and —OR

wherein R is a hydrocarbyl group having 1 to 20 carbon atoms or in the case of the first formula hydrogen, or an omega-halo-or omega-ester-alpha-olefin, with

(2) a hydrocarbyl monoolefin having at least three carbon atoms in the presence of a catalyst composition comprising

(a) at least one neutral carbene complex having the formula

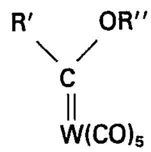

wherein R′ is selected from phenyl or an alkyl group having 1 to 10 carbon atoms and R″ is an alkyl radical having 1 to 10 carbon atoms; and

(b) a metal component comprising $WCl_6$ or a mixture of $SnCl_4$ and either $SiCl_4$ or $GeCl_4$,

and then, in case of (1) being said omega-halo-alpha-olefin, converting the halide to an ester, characterized in that an internal monoolefin is used as said hydrocarbyl monoolefin.

2. The process of claim 1 characterized in that the olefinic hydrocarbyl portion of said functional monoolefin has the formula

$$R'''—CH=CH(CH_2)_n—$$

wherein n is in the range of 2 to 10 and R‴ is hydrogen or an alkyl radical having 1 to 20 carbon atoms.

3. The process of claim 1 or 2 characterized in that said functional monoolefin is selected from 9-decenyl acetate, oleyl acetate, 1,18 - diacetoxy - 9 - octadecene, 9-decenyl formate, 9-decenyl propionate, 5-pentenyl acetate and methyl - 10 - undecenoate.

4. The process of any of claims 1 to 3 characterized in that said internal olefin comprises a symmetrical olefin.

5. The process of claim 4 characterized in that said internal olefin is selected from 5-decene, 2-butene, 3-hexene, 4-octene and 7-tetradecene.

6. The process of claim 5 characterized in that said internal olefin is 5-decene produced by the self-disproportionation of 1-hexene in presence of the same catalyst specified for the reaction of the functional olefin.

7. The process of any of the preceding claims characterized in that said functional olefin reactant consists essentially of the trans isomer of 1,18 - diacetoxy - 9 - octadecene.

8. The process of any of claims 1 to 6 characterized in that said functional olefin is a symmetrical difunctional olefin consisting essentially of the trans isomer.

9. The process of any of claims 1 to 6 characterized in that said internally unsaturated olefin, said functional monoolefin and said hydrocarbyl monoolefin are related as follows

| Internally unsaturated olefin | Functional monoolefin | Hydrocarbyl monoolefin |
|---|---|---|
| 9-dodecenyl acetate | 9-decenyl acetate | 3-hexene |
| 9-tridecenyl acetate | 9-decenyl acetate | trans-4-octene |
| 9-hexadecenyl acetate | 9-decenyl acetate | 7-tetradecene. |

10. The process for preparing 9-tetradecenyl formate obtained by reacting the acetate with water to

obtain the corresponding alcohol and then reacting the alcohol with formic acid to obtain said formate, characterized in that said 9-tetradecenyl acetate is obtained by reacting 5-decene with 9-decenyl acetate according to the process of claim 1.

11. The process for preparing 9-hexadecenyl alcohol obtained by reacting the acetate with water to obtain said alcohol, characterized in that said 9-hexadecenyl acetate is obtained by reacting 7-tetradecene with 9-decenyl acetate according to the process of claim 1.

12. The process for preparing 9-tetradecenyl alcohol obtained by reacting the acetate with water to obtain said alcohol, characterized in that said 9-tetradecenyl acetate is obtained by reacting 5-decene with 9-decenyl acetate according to the process of claim 1.

13. The process for preparing 9-tetradecenal obtained by reacting the acetate with water to obtain the alcohol and further oxidizing the alcohol to said aldehyde, characterized in that said 9-tetradecenyl acetate is obtained by reacting 5-decene with 9-decenyl acetate according to the process of claim 1.

14. The process of claim 1, wherein said omega-halo-alpha-olefin and omega-ester-alpha-olefin, respectively, has been obtained by hydrohalogenating an alpha, omega diene to obtain said omega-halo-alpha-olefin and then, optionally, converting the halo olefin to an ester olefin.

15. The process of claim 14, wherein HBr is used in the hydrohalogenation.

16. The method of claim 14 or 15 characterized in that 7-dodecenyl formate is produced from 1,7-octadiene or 9-tetradecenyl acetate is produced from 1,9-decadiene.

17. A composition suitable as insecticide characterized by containing as active ingredient 9-dodecenyl acetate having a trans/cis ratio greater than 0.25/1.

18. The composition of claim 17 characterized in that said trans/cis molar ratio is in the range of 1/1 to 3/1; in particular about 2/1.

19. A composition suitable as insecticide containing as active ingredient an admixture of the cis and trans isomers of 9-tetradecenyl formate characterized in that the molar ratio of trans to cis is in the range of 6/1 to 1/1.

20. The composition of claim 19 characterized in that said molar ratio of trans to cis is in the range of 5/1 to 2/1, in particular about 2/1.

21. A composition suitable as insecticide characterized by containing as active ingredient a mixture of the cis and trans isomers of 9-tetradecenal or an admixture of the cis and trans isomers of 9-hexadecenyl alcohol wherein the trans to cis ratio is about 2/1.

22. A composition suitable as insecticide containing as active ingredient an admixture of the cis and trans isomers of 9-tetradecenyl acetate characterized in that the molar ratio of trans to cis is in the range of 6/1 to 1/1.

23. The composition of claim 22 characterized in that said molar ratio of trans to cis is about 5/1.

24. The composition of claim 22 characterized in that said molar ratio of trans to cis is about 2/1.

25. The use of the composition of claim 17 or 18 for controlling the Western Pine Shoot Borer by disrupting the mating thereof.

26. The use of the composition of any of claims 19 to 24 for controlling Heliothis spp. by disrupting the mating thereof; in particular wherein said Heliothis species is Heliothis zea or Heliothis virescens.

**Patentansprüche**

1. Verfahren zur Herstellung eines intern ungesättigten Olefins mit einer sauerstoffhaltigen funktionellen Gruppe, erhalten durch Umsetzen von

(1) einem funktionellem Monoolefin mit mindestens einer terminalen funktionellen Gruppe, die unter folgenden Formeln ausgewählt ist

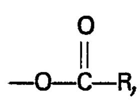 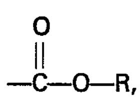 und —OR

worin R einen Hydrocarbylrest mit 1 bis 20 Kohlenstoffatomen oder im Fall der ersten Formel Wasserstoff bedeutet, oder einem omege-Halogen- oder omega - Ester - alpha - olefin, mit

(2) einem Hydrocarbylmonoolefin mit mindestens drei Kohlenstoffatomen in Gegenwart einer Katalysatorzusammensetzung, enthaltend

(a) mindestens einen neutralen Carbenkomplex der Formel

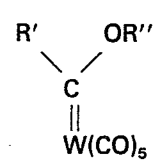

worin R′ ausgewählt ist unter Phenyl oder einem Alkylrest mit 1 bis 10 Kohlenstoffatomen und R″ einen Alkylrest mit 1 bis 10 Kohlenstoffatomen bedeutet; und

(b) eine Metallkomponente, die $WCl_6$ oder ein Gemisch aus $SnCl_4$ und entweder $SiCl_4$ oder $GeCl_4$ enthält,

und anschliessend für den Fall, dass (1) ein omega-Halogen-alpha-olefin ist, das Halogenid in einen Ester überführt, dadurch gekennzeichnet, dass ein internes Monoolefin als Hydrocarbylmonoolefin verwedent wird.

2. Verfahren nach Anspruch 1, dadurch gekennzeichnet, dass der olefinische Hydrocarbylteil des funktionellen Monoolefins folgende Formel aufweist

$$R'''—CH=CH(CH_2)_n—$$

worin n im Bereich von 2 bis 10 liegt und R''' Wasserstoff oder einen Alkylrest mit 1 bis 20 Kohlenstoffatomen bedeutet.

3. Verfahren nach Anspruch 1 oder 2, dadurch gekennzeichnet, dass das funktionelle Monoolefin ausgewählt ist unter 9-Decenylacetate, Oleylacetate, 1,18 - Diacetoxy - 9 - octadecen, 9 - Decenylformiat, 9 - Decenylpropionat, 5 - Pentenylacetat und Methyl - 10 - undecenoat.

4. Verfahren nach einem der Ansprüche 1 bis 3, dadurch gekennzeichnet, dass das interne Olefin ein symmetrisches Olefin umfasst.

5. Verfahren nach Anspruch 4, dadurch gekennzeichnet, dass das interne Olefin ausgewählt ist unter 5-Decen, 2-Buten, 3-Hexen, 4-Octen und 7-Tetradecen.

6. Verfahren nach Anspruch 5, dadurch gekennzeichnet, dass es sich beim internen Olefin um 5-Decen handelt, das durch Selbstdisproportionierung von 1-Hexen in Gegenwart des gleichen Katalysators, wie er für die Umsetzung des funktionellen Olefins angegeben ist, hergestellt worden ist.

7. Verfahren nach einem der vorstehenden Ansprüche, dadurch gekennzeichnet, dass der funktionelle Olefinreaktant im wesentlichen aus dem trans-Isomeren von 1,18 - Diacetoxy - 9 - octadecen besteht.

8. Verfahren nach einem der Ansprüche 1 bis 6, dadurch gekennzeichnet, dass es sich bei dem funktionellen Olefin um ein symmetrisches difunktionelles Olefin handelt, das im wesentlichen aus dem trans-Isomeren besteht.

9. Verfahren nach einem der Ansprüche 1 bis 6, dadurch gekennzeichnet, dass das intern ungesättigte Olefin, das funktionelle Monoolefin und das Hydrocarbylmonoolefin in folgendem Zusammenhang stehen:

| intern ungesättigtes Olefin | funktionelles Monoolefin | Hydrocarbyl-monoolefin |
|---|---|---|
| 9-Dodecenylacetate | 9-Decenylacetat | 3-Hexen |
| 9-Tridecenylacetate | 9-Decenylacetat | trans-4-Octen |
| 9-Hexadecenylacetat | 9-Decenylacetat | 7-Tetradecen |

10. Verfahren zur Herstellung von 9-Tetradecenylformiat, erhalten durch Umsetzen des Acetats mit Wasser unter Bildung des entsprechenden Alkohols und anschliessende Umsetzung des Alkohols mit Ameisensäure unter Bildung des Formiats, dadurch gekennzeichnet, dass das 9 - Tetradecenylacetat gemäss dem Verfahren von Anspruch 1 durch Umsetzung von 5-Decen mit 9 - Decenylacetate erhalten wird.

11. Verfahren zur Herstellung von 9-Hexadecenylalkohol, erhalten durch Umsetzung des Acetats mit Wasser unter Bildung des Alkohols, dadurch gekennzeichnet, dass das 9 - Hexadecenylacetat gemäss dem Verfahren von Anspruch 1 durch Umsetzung von 7 - Tetradecen mit 9 - Decenylacetat erhalten wird.

12. Verfahren zur Herstellung von 9-Tetradecenylalkohol, erhalten durch Umsetzung des Acetats mit Wasser unter Bildung des Alkohols, dadurch gekennzeichnet, dass das 9 - Tetradecenylacetat gemäss dem Verfahren von Anspruch 1 durch Umsetzung von 5-Decen mit 9 - Decenylacetat erhalten wird.

13. Verfahren zur Herstellung von 9-Tetradecenal, erhalten durch Umsetzung des Acetats mit Wasser unter Bildung des Alkohols und anschliessende Oxidation des Alkohols zum Aldehyd, dadurch gekennzeichnet, dass das 9 - Tetradecenylacetat gemäss dem Verfahren von Anspruch 1 durch Umsetzung von 5-Decen mit 9 - Decenylacetat erhalten wird.

14. Verfahren nach Anspruch 1, wobei das omega-Halogen-alpha-olefin bzw. das omega-Ester-alpha-olefin durch Hydrohalogenierung eines alpha-omega-Diens unter Bildung des omega-Halogen-alpha-olefins und anschliessende Umwandlung des Halogenolefins zu einem Esterolefin erhalten worden ist.

15. Verfahren nach Anspruch 14, wobei HBr bei der Hydrohalogenierung verwendet wird.

16. Verfahren nach Anspruch 14 oder 15, dadurch gekennzeichnet, dass 7 - Dodecenylformiat aus 1,7 - Octadien oder 9 - Tetradecenylacetat aus 1,9 - Decadien gebildet wird.

17. Als Insektizid geeignete Zusammensetzung, dadurch gekennzeichnet, dass sie als aktiven Bestandteil 9 - Dodecenylacetat mit einem trans/cis Verhältnis von mehr als 0,25/1 enthält.

18. Zusammensetzung nach Anspruch 17, dadurch gekennzeichnet, dass das trans/cis - Molverhältnis im Bereich von 1/1 bis 3/1 liegt und insbesondere etwa 2/1 beträgt.

19. Als Insektizid geeignete Zusammensetzung, enthaltend als aktiven Bestandteil ein Gemisch der cis- und trans-Isomeren von 9 - Tetradecenylformiat, dadurch gekennzeichnet, dass das Molverhältnis von trans zu cis im Bereich von 6/1 bis 1/1 liegt.

20. Zusammensetzung nach Anspruch 19, dadurch gekennzeichnet, dass das Molverhältnis von trans zu cis im Bereich von 5/1 bis 2/1 liegt und insbesondere etwa 2/1 beträgt.

21. Als Insektizid geeignete Zusammensetzung, dadurch gekennzeichnet, dass sie als aktiven Bestandteil ein Gemisch der cis- und trans-Isomeren von 9 - Tetradecenal oder ein Gemisch der cis- und trans-Isomeren von 9 - Hexadecenylalkohol enthält, wobei das Verhältnis von trans zu cis etwa 2/1 beträgt.

22. Als Insektizid geeignete Zusammensetzung, enthaltend als aktiven Bestandteil ein Gemisch der cis- und trans-Isomeren von 9 - Tetradecenylacetat, dadurch gekennzeichnet, dass das Molverhältnis von trans zu cis im Bereich von 6/1 bis 1/1 liegt.

23. Zusammensetzung nach Anspruch 22, dadurch gekennzeichnet, dass das Molverhältnis von trans zu cis etwa 5/1 beträgt.

24. Zusammensetzung nach Anspruch 22, dadurch gekennzeichnet, dass das Molverhältnis von trans zu cis etwa 2/1 beträgt.

25. Verwendung der Zusammensetzung nach Anspruch 17 oder 18 zur Bekämpfung von Encosoma sonomana durch Unterbrechung von deren Paarung.

26. Verwendung der Zusammensetzung nach einem der Ansprüche 19 bis 24 zur Bekämpfung von Heliothis spp. durch Unterbrechung von deren Paarung; wobei es sich bei der Heliothis - Spezies insbesondere um Heliothis zea oder Heliothis virescens handelt.

**Revendications**

1. Procédé de préparation d'une oléfine à insaturation interne ayant un groupe fonctionnel oxygéné, obtenue en faisant réagir:

(1) une monooléfine fonctionnelle ayant au moins un groupe fonctionnel terminal choisi parmi les formules:

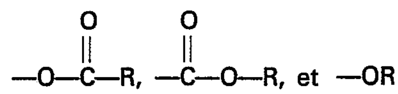

dans lesquelles R est un groupe hydrocarbyle en $C_1$ à $C_{20}$, ou dans le cas de la première formule, un atome d'hydrogène, ou une oméga-halo ou une oméga - ester - alpha - oléfine, avec

(2) une hydrocarbyl monooléfine ayant au moins trois atomes de carbone en présence d'une composition de catalyseur comprenant

(a) au moins un complexe de carbène neutre répondant à la formule:

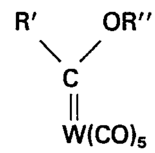

dans laquelle R′ est choisi parmi un groupe phényle ou un groupe alkyle en $C_1$ à $C_{10}$ et R″ est un radical alkyle en $C_1$ à $C_{10}$; et

(b) un constituant métallique comprenant $WCl_6$ ou un mélange de $SiCl_4$ et soit de $SiCl_4$, soit de $GeCl_4$, puis, dans le cas où (1) est cet oméga - halo - alpha - oléfine, en transformant l'halogènure en ester, caractérisé en ce qu'on utilise une monooléfine interne comme cet hydrocarbylmonooléfine.

2. Procédé selon la revendication 1, caractérisé en ce que la partie hydrocarbyle de cette monooléfine fonctionnelle répond à la formule:

$$R'''—CH=CH(CH_2)_n—$$

dans laquelle n est dans l'intervalle de 2 à 10 et R‴ est un atome d'hydrogène ou un radical alkyle en $C_1$ à $C_{20}$.

3. Procédé selon les revendications 1 ou 2, caractérisé en ce que cette monooléfine fonctionnelle est choisie parmi l'acétate de 9 - décényle, l'acétate d'oléyle, le 1,18 - diacétoxy - 9 - octadécène, le formiate de 9 - décényle, le propionate de 9 - décényle, l'acétate de 5 - penténylе et le 10 - undécénoate de méthyle.

4. Procédé selon l'une quelconque des revendications 1 à 3, caractérisé en ce que cette oléfine interne comprend une oléfine symétrique.

5. Procédé selon la revendication 4, caractérisé en ce que cette oléfine interne est choisie parmi le 5-décène, le 2-butène, le 3-hexène, le 4-octène et le 7-tétradécène.

6. Procédé selon la revendication 5, caractérisé en ce que cette oléfine interne est le 5-décène préparé

par auto-dismutation du 1-hexène en présence du même catalyseur que celui indiqué pour la réaction de l'oléfine fonctionnelle.

7. Procédé selon l'une quelconque des revendications précédentes, caractérisé en ce que ce réactif d'oléfine fonctionnelle est essentiellement constitué de l'isomère trans du 1,18 - diacétoxy - 9 - octadécène.

8. Procédé selon l'une quelconque des revendications 1 à 6, caractérisé en ce que cette oléfine fonctionnelle est une oléfine difonctionnelle symétrique essentiellement constituée de l'isomère trans.

9. Procédé selon l'une quelconque des revendications 1 à 6, caractérisé en ce que cette oléfine à insaturation interne, cette monooléfine fonctionnelle et cette hydrocarbyl monooléfine sont reliées comme suit:

| Oléfine à insaturation interne | Monooléfine fonctionnelle | Hydrocarbyl monooléfine |
|---|---|---|
| acétate de 9-dodécényle | acétate de 9-décényle | 3-hexène |
| acétate de 9-tridécényle | acétate de 9-décényle | trans-4-octène |
| acétate de 9-hexadécényle | acétate de 9-décényle | 7-tétradécène |

10. Procédé de préparation du formiate de 9 - tétradécényle obtenu en faisant réagir l'acétate avec de l'eau pour obtenir l'alcool correspondant, puis en faisant réagir l'alcol avec l'acide formique pour obtenir ce formiate, caractérisé en ce que cet acétate de 9 - tétradécényle est obtenu en faisant réagir le 5-décène avec l'acétate de 9-décényle conformément au procédé de la revendication 1.

11. Procédé pour préparer l'alcool 9 - hexadécénylique obtenu en faisant réagir l'acétate avec de l'eau pour obtenir cet alcool, caractérisé en ce que cet acétate de 9 - hexadécényle est obtenu en faisant réagir le 7 - tétradécène avec l'acétate de 9 - décényle conformément au procédé de la revendication 1.

12. Procédé de préparation de l'alcool 9 - tétradécénylique obtenu en faisant réagir l'acétate avec de l'eau pour obtenir cet alcool, caractérisé en ce que cet acétate de 9 - tétradécényle est obtenu en faisant réagir du 5-décène avec de l'acétate de 9-décényle conformément au procédé de la revendication 1.

13. Procédé de préparation du 9 - tétradécénal obtenu en faisant réagir l'acétate avec de l'eau pour obtenir l'alcool et en oxydant encore l'alcool en cet aldéhyde, caractérisé en e que cet acétate de 9 - tétradécényle est obtenu en faisant réagir le 5-décène avec l'acétate de 9-décényle conformément au procédé de la revendication 1.

14. Procédé selon la revendication 1, dans lequel cette oméga - halo - alpha - oléfine et cette oméga - ester - alpha - oléfine, respectivement, ont été obtenues par hydrohalogénation d'un alpha, oméga diène pour obtenir cette oméga - halo - alpha - oléfine, puis, si on le désire, en transformatn l'halo oléfine en une ester oléfine.

15. Procédé selon la revendication 14, dans lequel on utilise dans l'hydrohalogénation du HBr.

16. Procédé selon les revendications 14 ou 15, caractérisé en ce que le formiate de 7 - dodécényle est préparé à partir du 1,7 - octadiène ou en ce que l'acétate de 9 - tétradécényle est préparé à partir du 1,9-décadiène.

17. Composition convenant comme insecticide, caractérisée en ce qu'elle contient comme ingrédient actif un acétate de 9 - dodécényle ayant un rapport trans/cis supérieur à 0,25/1.

18. Composition selon la revendication 17, caractérisée en ce que ce rapport molaire trans/cis est dans l'intervalle de 1/1 à 3/1; en particulier d'environ 2/1.

19. Composition convenant comme insecticide, contenant comme ingrédient actif un mélange des isomères cis et trans du formiate de 9 - tétradécényle, caractérisée en ce que le rapport molaire de l'isomère trans à l'isomère cis est dans l'intervalle de 6/1 à 1/1.

20. Composition selon la revendication 19, caractérisée en ce que ce rapport molaire de l'isomère trans à l'isomère cis est dans l'intervalle de 5/1 à 2/1, en particulier d'environ 2/1.

21. Composition convenant comme insecticide, caractérisée en ce qu'elle contient comme ingrédient actif un mélange des isomères cis et trans du 9 - tétradénal ou un mélange des isomères cis et trans de l'alcool 9 - hexadécénylique dans lequel le rapport de l'isomère trans à l'isomère cis est d'environ 2/1.

22. Composition convenant comme insecticide, contenant comme ingrédient actif un mélange des isomères cis et trans de l'acétate de 9 - tétradécényle, caractérisée en ce que le rapport molaire de l'isomère trans à l'isomère cis est dans l'intervalle de 6/1 à 1/1.

23. Composition selon la revendication 22, caractérisée en ce que ce rapport molaire de l'isomère trans à l'isomère cis est d'environ 5/1.

24. Composition selon la revendication 22, caractérisée en ce que ce rapport molaire de l'isomère trans à l'isomère cis est d'environ 2/1.

25. Utilisation de la composition des revendications 17 ou 18 pour lutter contre l'insecte térébrant des jeunes pousses du pin à bois lourd en interrompant son accouplement.

26. Utilisation de la composition selon l'une quelconque des revendications 19 à 24 pour lutter contre Héliothis spp. en interrompant son accouplement; en particulier dans laquelle cette espèce Heliothis est Heliothis zea ou Heliothis virescens.